(19) Europäisches Patentamt
European Patent Office
Office européen des brevets

(11) Publication number: **0 029 092**
**B1**

(12) # EUROPEAN PATENT SPECIFICATION

(45) Date of publication of patent specification: **05.01.83**

(51) Int. Cl.³: **C 07 C 49/743, C 07 D 307/93, C 07 C 45/61**

(21) Application number: **80105103.8**

(22) Date of filing: **27.08.80**

(54) **Cis-(1R,2S)-3,3-dimethyl-2-((4S)-3-methyl-2-(2-propynyl)cyclopent-2-en-1-on-4-yloxyhydroxymethyl)-cyclopropanecarboxylic lactone and method of producing (S)-4-hydroxy-3-methyl-2-(2-propynyl)-cyclopent-2-en-1-on.**

(30) Priority: **06.09.79 JP 114771/79**
**06.09.79 JP 114772/79**

(43) Date of publication of application:
**27.05.81 Bulletin 81/21**

(45) Publication of the grant of the patent:
**05.01.83 Bulletin 83/1**

(84) Designated Contracting States:
**BE CH DE FR GB IT LI NL**

(56) References cited:
**EP - A - 0 004 493**
**DE - A - 2 348 930**
**DE - A - 2 738 647**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Itaya, Nobushige**
**5-3-101, Ryodocho**
**Nishinomiya-shi (JP)**
Inventor: **Matsuo, Noritada**
**29-2, Aza Yamamichi**
**Minamino, Itami-shi (JP)**

(74) Representative: **PATENTANWÄLTE HENKEL - KERN - FEILER - HÄNZEL**
**Möhlstrasse 37**
**D-8000 München 80 (DE)**

**EP 0 029 092 B1**

**Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).**

Courier Press, Leamington Spa, England.

Cis-(1R,2S)-3,3-dimethyl-2-((4S)-3-methyl-2-(2-propynyl)cyclopent-2-en-1-on-4-yloxyhydroxy-methyl)-cyclopropanecarboxylic lactone and method of producing (S)-4-hydroxy-3-methyl-2-(2-propynyl)-cyclopent-2-en-1-on

The present invention relates to a method for producing (S)-4-hydroxy-3-methyl-2-(2-propynyl)cyclopent-2-en-1-one of the formula (I),

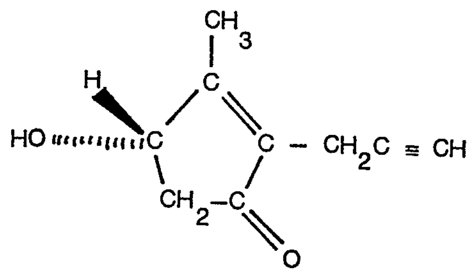

(I)

More particularly, it relates to a method for producing the compound of the formula (I) which comprises solvolyzing *cis*-(1R, 2S)-3,3-dimethyl-2-[(4S)-3-methyl-2-(2-propynyl)cyclopent-2-en-1-on-4-yloxyhydroxymethyl]-cyclopropanecarboxylic lactone of the formula (II),

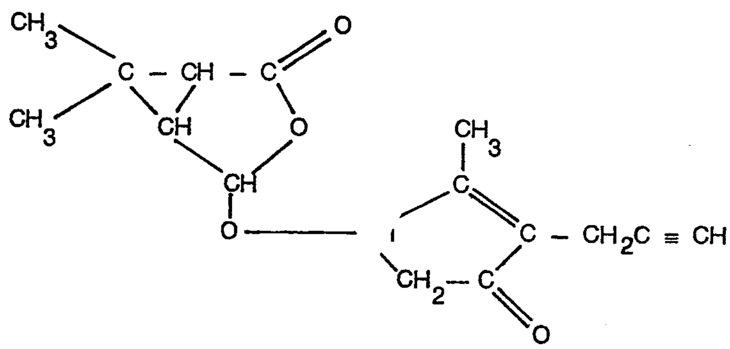

(II)

with a compound of the formula (I'

$$R_1OH \qquad (III)$$

wherein $R_1$ is a hydrogen atom or a $C_1$—$C_4$ alkyl group, in an alkaline condition or in the presence of an acidic catalyst (preferably in the presence of an acidic catalyst); *cis*-(1R, 2S)-3,3-dimethyl-2-[(4S)-3-methyl-2-(2-propynyl)cyclopent-2-en-1-on-4-yloxyhydroxymethyl]-cyclopropanecarboxylic lactone of the formula (II), and a method for producing the lactone of the formula (II) which comprises reacting *cis*-(1R, 2S)-3,3-dimethyl-2-(dihydroxymethyl)cyclopropanecarboxylic lactone of the formula (IV),

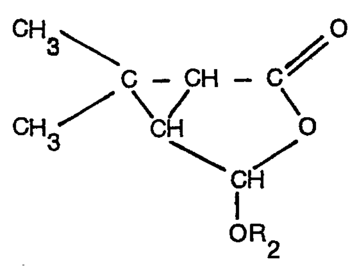

(IV)

wherein $R_2$ is a hydrogen atom or a $C_1$—$C_4$ alkyl group, with (RS)-4-hydroxy-3-methyl-2-(2-propynyl)-cyclopent-2-en-1-one in the presence of an acidic catalyst while removing a compound of the formula (V),

$$R_2OH \qquad (V)$$

wherein $R_2$ is as defined above, produced by the reaction as by-product from the reaction system (hereinafter the reaction of this method is referred to as condensation); and crystallizing the resulting

lactone (II) in the presence or absence of a solvent and separating the crystal from the mother liquor, whereby the lactone of the formula (II) can be obtained easily.

As is well known, esters resulting from 4-hydroxy-3-methyl-2-(2-propynyl)cyclopent-2-en-1-one and various acids including chrysanthemic acid have a strong insecticidal activity like allethrin. Particularly, it is well known that the ester resulting from 4-hydroxy-3-methyl-2-(2-propynyl)cyclopent-2-en-1-one and 2,2,3,3-tetramethylcyclopropanecarboxylic acid has very strong knock-down and lethal effects (British Pat. 1394416). The ester resulting from (S)-4-hydroxy-3-methyl-2-(2-propynyl)-cyclopent-2-en-1-one and 2,2,3,3-tetramethylcyclopropanecarboxylic acid has an insecticidal activity of about twice as strong as that of the corresponding racemate.

As a result of a long-term study on the production of (S)-4-hydroxy-3-methyl-2-(2-propynyl)-cyclopent-2-en-1-one of the formula (I), the inventors found a novel and very advantageous method for producing the compound of the formula (I). The inventors made a further study on the important intermediate for this method and production thereof, and attained to the present invention.

Next, the method of the present invention will be illustrated specifically.

Production of (S)-4-hydroxy-3-methyl-2-(2-propynyl)cyclopent-2-en-1-one of the formula (I)

In applying solvolysis to *cis*-(1R, 2S)-3,3-dimethyl-2-[(4S)-3-methyl-2-(2-propynyl)cyclopent-2-en-1-on-4-yloxyhydroxymethyl]cyclopropanecarboxylic lactone of the formula (II), when the solvolysis is carried out in an alkaline condition (pH: not more than 12), it proceeds in the form of the hydrolysis of the lactone ring, and the common mild hydrolysis conditions may be applied thereto. That is, the hydrolysis is carried out by reacting the lactone with a compound of the formula (III); $R_1OH$ at 0° to 50°C while controlling the pH of the reaction system so as not to exceed 12 using an inorganic base (e.g. sodium hydroxide, potassium hydroxide, sodium carbonate, potassium carbonate, sodium hydrogencarbonate, calcium carbonate). The amount of the compound (III) used for reaction is equivalent or more to *cis* - (1R, 2S) - 3,3 - dimethyl - 2 - [(4S) - 3 - methyl - 2 - (2 - propynyl)cyclopent - 2 - en - 1 - on - 4 - yloxyhydroxymethyl]cyclopropanecarboxylic lactone.

In the present invention, however, acid solvolysis (pH: not more than 5) is more desirable, and in this case, the solvolysis is easily achieved by reacting the lactone with the compound of the formula (III); $R_1OH$ in a solvent at from room temperature (e.g. 20°C) to the boiling point of the solvent for 0.5 to 4 hours in the presence of a catalytic amount of acidic catalyst. The solvent includes water, a $C_1$—$C_4$ alcohol itself of the formula (III) and mixtures thereof. The acidic catalyst includes for example organic acids [e.g. oxalic acid, formic acid, halogenated acetic acids (e.g. trichloroacetic acid, trifluoroacetic acid), organic sulfonic acids (e.g. p-toluenesulfonic acid)], mineral acids (e.g. hydrochloric acid, sulfuric acid, phosphoric acid, perchloric acid, nitric acid) and hydrogensulfates (e.g. potassium hydrogensulphate). The amount of the compound (III) used for reaction is equivalent or more to *cis* - (1R, 2S)-3,3 - dimethyl - 2 - [(4S) - 3 - methyl - 2 - (2 - propynyl)cyclopent - 2 - en - 1 - on - 4 - yloxyhydroxymethyl]cyclopropanecarboxylic lactone.

After completion of the reaction, the reaction solution is cooled to room temperature (e.g. 20°C) or to less than that (to 10°C) and neutralized, for example, with an aqueous sodium hydrogencarbonate solution. Thereafter, the solution is extracted, after concentrated or as it is, with an inert solvent, and the extract is concentrated or, if necessary, distilled to obtain the objective (S)-4-hydroxy-3-methyl-2-(2-propynyl)cyclopent-2-en-1-one of the formula (I). The inert solvent includes for example hydrocarbon solvents (e.g. benzene, toluene, n-hexane, n-heptane), halogenated hydrocarbon solvents (e.g. dichloromethane, chloroform, carbon tetrachloride), ether solvents (e.g. diisopropyl ether, tetrahydrofuran) and ester solvents (e.g. ethyl acetate). In the solvolysis with $C_1$—$C_4$ alcohols, *cis*-(1R, 2S)-3,3-dimethyl-2-($C_1$—$C_4$ alkoxyhydroxymethyl)cyclopropanecarboxylic lactone is recovered, and in the solvolysis with water, *cis*-(1R, 2S)-3,3-dimethyl-3-(dihydroxymethyl)cyclopropanecarboxylic lactone is recovered.

Production of *cis*-(1R, 2S)-3,3-dimethyl-2-[(4S)-3-methyl-2-(2-propynyl)cyclopent-2-en-1-on-4-yloxyhydroxymethyl]cyclopropanecarboxylic lactone of the formula (II)

One mole of racemic (RS)-4-hydroxy-3-methyl-2-(2-propynyl)cyclopent-2-en-1-one which may contain the optically active isomer in part is allowed to react with 0.5 to 1.5 moles of *cis*-(1R, 2S)-3,3-dimethyl-2-(dihydroxymethyl)cyclopropanecarboxylic lactone or *cis*-(1R, 2S)-3,3-dimethyl-2-($C_1$—$C_4$ alkoxyhydroxymethyl)cyclopropanecarboxylic lactone without solvent or in an inert solvent in the presence of a catalytic amount of an acidic catalyst. The inert solvent includes for example hydrocarbon solvents (e.g. benzene, toluene, n-hexane, n-heptane), halogenated hydrocarbon solvents (e.g. dichloromethane, chloroform, carbon tetrachloride), ether solvents (e.g. diisopropyl ether, tetrahydrofuran) and ester solvents (e.g. ethyl acetate). These solvents may be used alone or in combination. The acidic catalyst includes for example organic acids [e.g. oxalic acid, formic acid, halogenated acetic acids (e.g. trichloroacetic acid, trifluoroacetic acid), organic sulfonic acids (e.g. p-toluenesulfonic acid)], mineral acids (e.g. hydrochloric acid, sulfuric acid, phosphric acid, perchloric acid, nitric acid) and hydrogensulfates (e.g. potassium hydrogensulfate). This condensation is achieved by removing water or a $C_1$—$C_4$ alcohol of the formula (V); $R_2OH$ produced as by-product from the reaction system, for example, by distillation under reduced pressure by heating, by azeotropic distillation under

atmospheric pressure, or by the use of dehydrating or alcohol-removing agents such as molecular sieves. In the case of removal by azeotropic distillation, the condensation proceeds rapidly, coming to an end in 30 minutes to 4 hours in general, but it may be regarded as coming to an end at the time when the temperature of the distillate has reached the boiling point of the solvent (azeotropic temperature when a mixed solvent is used). In the case of removal by molecular sieves, the condensation proceeds even in the vicinity of room temperature, and it comes to an end in 30 minutes to 24 hours in general, although the temperature and time depend also upon the amounts of the reagents.

After completion of the reaction, the reaction solution is cooled to room temperature (e.g. 20°C) or to less than that (to 10°C), neutralized with an aqueous sodium hydrogencarbonate or sodium hydroxide solution or an amine, washed and concentrated to obtain *cis* - (1R, 2S) - 3,3 - dimethyl - 2 - [(4S) - 3 - methyl - 2 - (2 - propynyl)cyclopent - 2 - en - 1 - on - 4 - yloxyhydroxymethyl]-cyclopropanecarboxylic lactone and its diastereoisomer as a stable mixture. This desired lactone can be isolated in a pure form by crystallizing from the mixture without solvent or from the foregoing inert organic solvent and separating the crystal from the mother liquor.

The present invention will be illustrated in more detail with reference to the following examples, which are not however to be interpreted as limiting the invention thereto.

### Example 1

(RS)-4-Hydroxy-3-methyl-2-(2-propynyl)cyclopent-2-en-1-one (1.88 g) and *cis*-(1R, 2S)-3,3-dimethyl-2-(dihydroxymethyl)cyclopropanecarboxylic lactone (1.9 g, described in Japanese Patent Publication No. 14563/1972) derived from d-cis-chrysanthemic acid were added to benzene (40 ml), and a catalytic amount of p-toluenensulfonic acid is further added thereto. The mixture was placed in a still with a rectifying tower and heated. After removing water produced by the reaction as benzene/water azeotropic mixture, the reaction was continued for further 30 minutes while distilling out benzene little by little. After cooling, the reaction solution was neutralized with a dilute aqueous sodium hydroxide solution, washed and separated into organic and aqueous layers. The organic layer was concentrated to obtain 2.8 g of an oily product. The oily product was crystallized from a mixture of ether (1 ml) and diisopropyl ether (10 ml). This crystal was separated from the mother liquor to obtain 0.7 g of *cis*-(1R, 2S)-3,3-dimethyl-2-[(4S)-3-methyl-2-(2-propynyl)cyclopent-2-en-1-on-4-yloxy-hydroxymethyl]cyclopropanecarboxylic lactone.

Melting point: 139°—140°C
Optical rotation: $[\alpha]_D^{25} = -76.0°$
(c = 1.14%, $CHCl_3$)

### Example 2

*Cis* - (1R, 2S) - 3,3 - dimethyl - 2 - [(4S) - 3 - methyl - 2 - (2 - propynyl)cyclopent - 2 - en - 1 - on - 4 - yloxyhydroxymethyl]cyclopropanecarboxylic lactone (0.58 g) obtained in Example 1, methanol (30 g) and a catalytic amount of p-toluenesulfonic acid were mixed and heated under reflux for 30 minutes. After cooling, the reaction solution was neutralized with a small amount of an aqueous sodium hydrogencarbonate solution, and methanol was removed under reduced pressure at room temperature. A small amount of water was added to the residue which was then extracted with ethyl acetate. The extract was concentrated and distilled to remove *cis*-(1R, 2S)-3,3-dimethyl-2-(methoxy-hydroxymethyl)cyclopropanecarboxylic lactone as a fraction having a boiling point of 45°—50°C/0.2 mmHg. The resulting residue (0.26 g) was further distilled to obtain 0.25 g of a fraction having a boiling point of 115°C/0.3 mmHg. It was found from NMR spectrum that this fraction was almost pure (S)-4-hydroxy-3-methyl-2-(2-propynyl)cyclopent-2-en-1-one.

Optical rotation: $[\alpha]_D^{25} = +20.0°$
(c = 2.0%, $CHCl_3$)

### Example 3

Reaction was carried out in the same manner as in Example 2 except that water (30 g) and a catalytic amount of conc. hydrochloric acid were used in place of methanol (30 g) and p-toluenesulfonic acid, respectively. After cooling, the reaction solution was neutralized with an aqueous sodium hydrogencarbonate solution and extracted with ethyl acetate. The extract was concentrated and distilled to obtain 0.26 g of (S)-4-hydroxy-3-methyl-2-(2-propynyl)cyclopent-2-en-1-one of the same quality as that of the compound obtained in Example 2.

### Example 4

Reaction was carried out in the same manner as in Example 1 except that *cis*-(1R, 2S)-3,3-dimethyl-2-(methoxyhydroxymethyl)cyclopropanecarboxylic lactone (2.1 g) was used in place of *cis*-(1R, 2S)-3,3-dimethyl-2-(dihydroxymethyl)cyclopropanecarboxylic lactone, and that methanol produced by the reaction was removed as a methanol/benzene azeotropic mixture. Thus, 2.6 g of a

concentrated oily product was obtained. The crystal (0.61 g) obtained from this oily product had the same quality as that of the crystal obtained in Example 2.

Reference Example

The (S)-2-methyl-3-(2-propynyl)cyclopent-2-en-4-on-1-yl 2,2,3,3-tetramethylcyclopropanecarboxylate ($[\alpha]_D = -7.2°$, c = (0.5, $CHCl_3$)) [Compound A] is obtained by reacting the present compound, (S)-4-hydroxy-3-methyl-2-(2-propynyl)cyclopent-2-en-1-one with 2,2,3,3-tetramethylcyclopropanecarbonyl chloride in the manner described in U.S. Patent No. 3876681.

And, the Compound A and reference compound were dissolved in kerosene to obtain an oil spray having the prescribed concentration of each compound. Ten northern house mosquito female adults (*Culex pipiens pallens*) and ten housefly adults (*Musca domestica*) were liberated in a (70 cm)³ glass chamber, and 0.7 ml of the above oil spray was sprayed into the chamber. Thereafter, the number of knocked-down insects was counted with the lapse of time, and the value of $KT_{50}$ was obtained from the average knock-down ratio of three replications according to the Finney's method.

| Test compound | Concentration (%) | $KT_{50}$ (sec) | |
|---|---|---|---|
| | | Housefly adult | Northern house mosquito female adult |
| Compound A | 0.1 | 84 | < 30 |
| | 0.05 | 130 | 38 |
| | 0.025 | 200 | 68 |
| Reference Compound * | 0.1 | 120 | 44 |
| | 0.05 | 210 | 85 |
| | 0.025 | 380 | 122 |

* Compound described in U.S. Patent No. 3876681.

## Claims

1. A method for producing (S)-4-hydroxy-3-methyl-2-(2-propynyl)cyclopent-2-en-1-one of the formula (I),

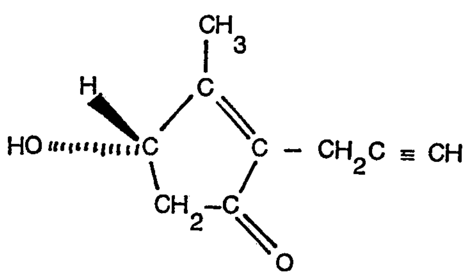

(I)

which comprises solvolyzing *cis*-(1R, 2S)-3,3-dimethyl-2-[(4S)-3-methyl-2-(2-propynyl)cyclopent-2-en-1-on-4-yloxyhydroxymethyl]cyclopropanecarboxylic lactone of the formula (II),

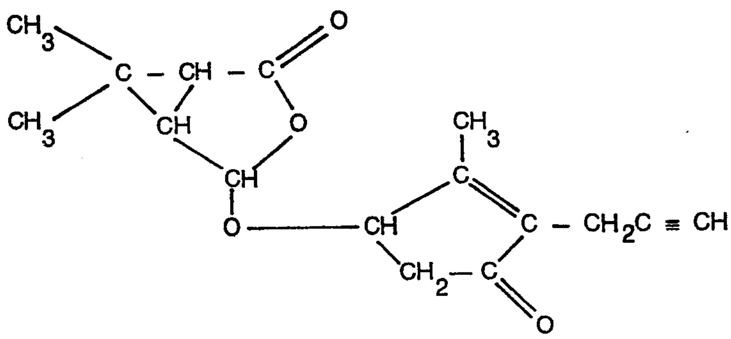

(II)

with a compound of the formula (III),

$$R_1OH \quad \text{(III)}$$

wherein $R_1$ is a hydrogen atom or a $C_1$—$C_4$ alkyl group, in an alkaline condition or in the presence of an acidic catalyst.

2. A method according to Claim 1, wherein the solvolysis is carried out by reacting the lactone of the formula (II) with the compound of the formula (III) in the presence of an acidic catalyst.

3. A method for producing *cis*-(1R, 2S)-3,3-dimethyl-2-[(4S)-3-methyl-2-(2-propynyl)cyclopent-2-en-1-on-4-yloxyhydroxymethyl]cyclopropanecarboxylic lactone of the formula (II),

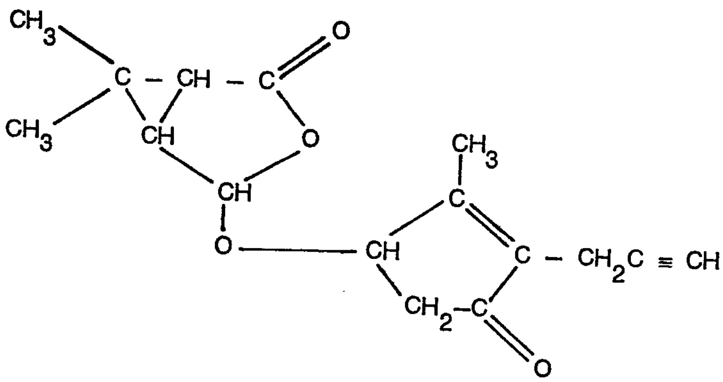

(II)

which comprises reacting *cis*-(1R, 2S)-3,3-dimethyl-2-(dihydroxymethyl)cyclopropanecarboxylic lactone of the formula (IV),

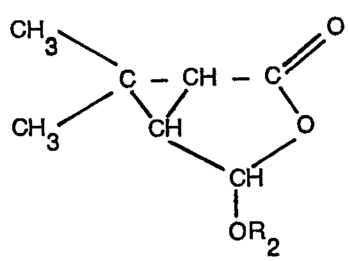

(IV)

wherein $R_2$ is a hydrogen atom or a $C_1$—$C_4$ alkyl group, with (R,S)-4-hydroxy-3-methyl-2-(2-propynyl)-cyclopent-2-en-1-one in the presence of an acidic catalyst while removing a compound of the formula (V),

$$R_2OH \quad \text{(V)}$$

wherein $R_2$ is as defined above, produced by the reaction as by-product from the reaction system, and crystallizing the resulting lactone of the formula (II) in the presence or absence of a solvent and separating the crystal from the mother liquor, whereby the lactone of the formula (II) can be obtained easily.

4. *cis*-(1R, 2S)-3,3-dimethyl-2-[(4S)-3-methyl-2-(2-propynyl)cyclopent-2-en-1-on-4-yloxyhydroxymethyl]cyclopropanecarboxylic lactone of the formula (II).

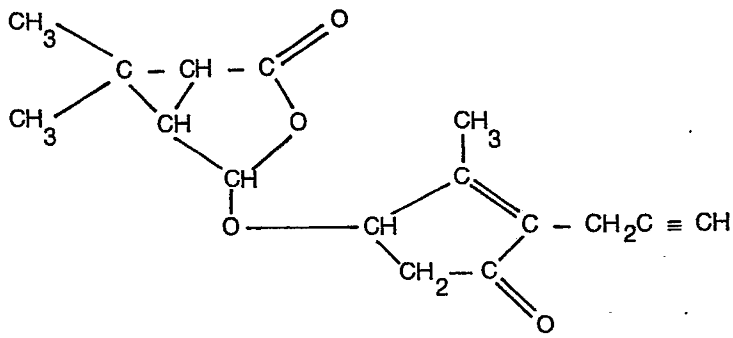

(II)

**Patentansprüche**

1. Verfahren zur Herstellung von (S)-4-Hydroxy-3-methyl-2-(2-propynyl)-cyclopent-2-en-1-on der Formel:

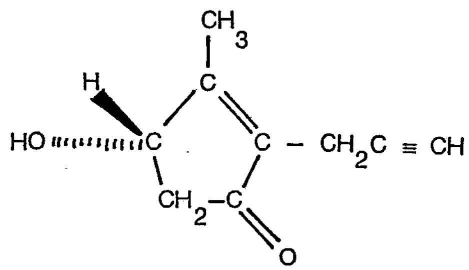

(I)

dadurch gekennzeichnet, daß man cis - (1R, 2S) - 3,3 - Dimethyl - 2 - [(4S) - 3 - methyl - 2 - (2 - propenyl) - cyclopent - 2 - en - 1 - on - 4 - yloxyhydroxymethyl] - cyclopropancarbonsäurelacton der Formel:

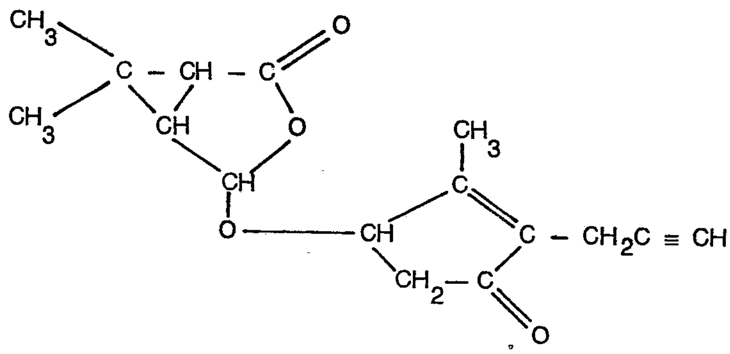

(II)

mit einer Verbindung der Formel:

$$R_1OH \qquad (III)$$

worin $R_1$ für ein Wasserstoffatom oder eine Alkylgruppe mit 1 bis 4 Kohlenstoffatom(en) steht, unter alkalischen Bedingungen oder in Gegenwart eines sauren Katalysators einer Solvolyse unterwirft.

2. Verfahren nach Anspruch 1, dadurch gekennzeichnet, daß man die Solvolyse durch Umsetzen des Lactons der Formel II mit der Verbindung der Formel III in Gegenwart eines sauren Katalysators durchführt.

3. Verfahren zur Herstellung von cis - (1R, 2S) - 3,3 - Dimethyl - 2 - [(4S) - 3 - methyl - 2 - (2 - propynyl) - cyclopent - 2 - en - 1 - on - 4 - yloxyhydroxymethyl] - cyclopropancarbonsäurelacton der Formel:

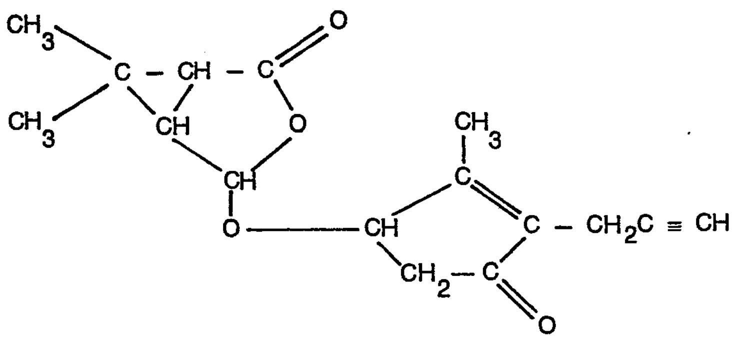

(II)

dadurch gekennzeichnet, daß man cis-(1R, 2S)-3,3-Dimethyl-2-(dihydroxymethyl)-cyclopropancarbonsäurelacton der Formel:

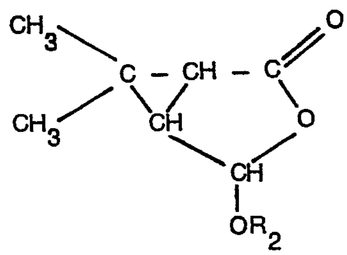

(IV)

worin $R_2$ für ein Wasserstoffatom oder eine Alkylgruppe mit 1 bis 4 Kohlenstoffatom(en) steht, mit (R,S)-4-Hydroxy-3-methyl-2-(2-propynyl)-cyclopent-2-en-1-on in Gegenwart eines sauren Katalysators reagieren läßt, gleichzeitig die bei der Reaktion als Nebenprodukt gebildete Verbindung der Formel:

$R_2OH$ (V)

worin $R_2$ die angegebene Bedeutung besitzt, aus dem Reaktionssystem entfernt und das erhaltene Lacton der Formel II in Gegenwart oder Abwesenheit eines Lösungsmittels zur Kristallisation bringt und schließlich die gebildeten Kristalle von der Mutterlauge abtrennt.

4. Cis - (1R, 2S) - 3,3 - dimethyl - 2 - [(4S) - 3 - methyl - 2 - (2 - propynyl) - cyclopent - 2 - en - 1 - on - 4 - yloxyhydroxymethyl] - cyclopropancarbonsäurelacton der Formel:

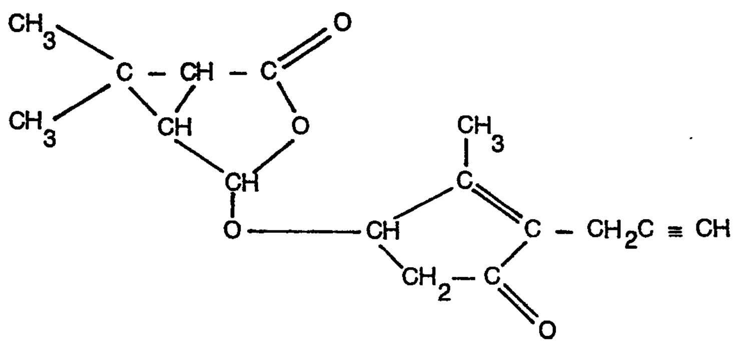

(II).

## Revendications

1. Procédé de préparation de la (S)-4-hydroxy-3-méthyl-2-(2-propynyl)cyclopent-2-ène 1-one de formule (I),

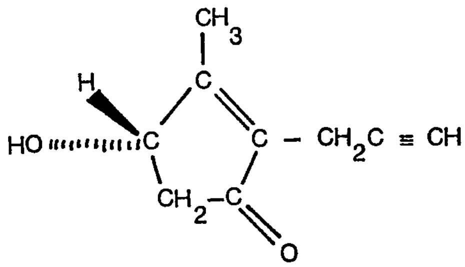

(I)

caractérisé en ce qu'on effectue la solvolyse de la lactone de l'acide *cis*-(1R, 2S)-3,3-diméthyl-2-[(4S)-3-méthyl-2-(2-propynyl)cyclopent-2-ène 1-one 4-yloxyhydroxyméthyl]cyclopropane carboxylique de formule (II),

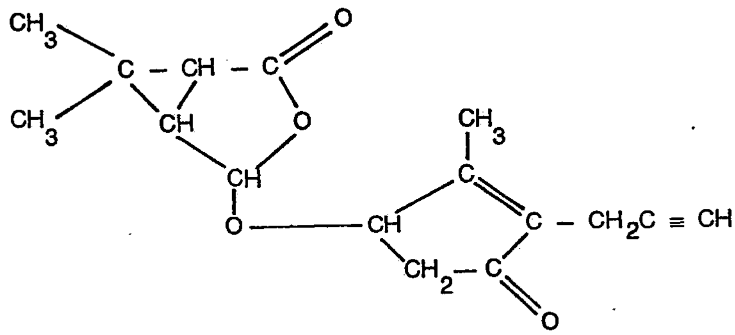

(II)

à l'aide d'un composé de formule (III),

$R_1OH$ (III)

dans laquelle $R_1$ est un atome d'hydrogène ou un groupe alcoyle en $C_1$—$C_4$, en présence d'un agent alcalin ou d'un catalyseur acide.

2. Procédé selon la revendication 1, dans lequel la solvolyse est effectuée par réaction de la lactone de formule (II) avec le composé de formule (III) en présence d'un catalyseur acide.

3. Procédé de préparation de la lactone de l'acide *cis*-(1R, 2S)-3,3-diméthyl-2-[(4S)-3-méthyl-2-(2-propynyl)cyclopent-2-ène 1-one 4-yloxyhydroxyméthyl]cyclopropane carboxylique de formule (II),

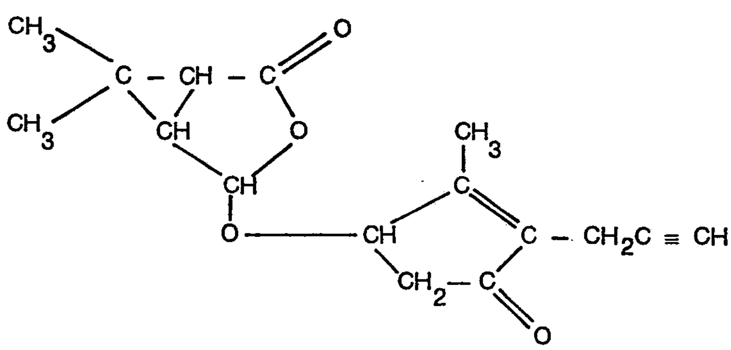

(II)

caractérisé en ce qu'on fait réagir la lactone de l'acide *cis*-(1R, 2S)-3,3-diméthyl-2-(dihydroxyméthyl)-cyclopropane carboxylique de formule (IV),

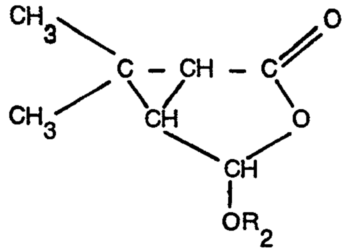

(IV)

dans laquelle $R_2$ est un atome d'hydrogène ou un groupe alcoyle en $C_1$—$C_4$, avec la (R,S)-4-hydroxy-3-méthyl-2-(2-propynyl)cyclopent-2-ène 1-one en présence d'un catalyseur acide tout en éliminant du mélange réactionnel un composé de formule (V),

$$R_2OH \qquad (V)$$

dans laquelle $R_2$ est tel que défini ci-dessus, composé qui se forme comme sous-produit de la réaction, et on cristallise la lactone formée de formule (II) en présence ou en absence d'un solvant et on sépare les cristaux de la liqueur mère, ce par quoi la lactone de formule (II) peut être obtenue aisément.

4. La lactone de l'acide *cis*-(1R, 2S)-3,3-diméthyl-2-[(4S)-3-méthyl-2-(2-propynyl)cyclopent-2-ène 1-one 4-yloxyhydroxyméthyl]cyclopropane carboxylique de formule (II),

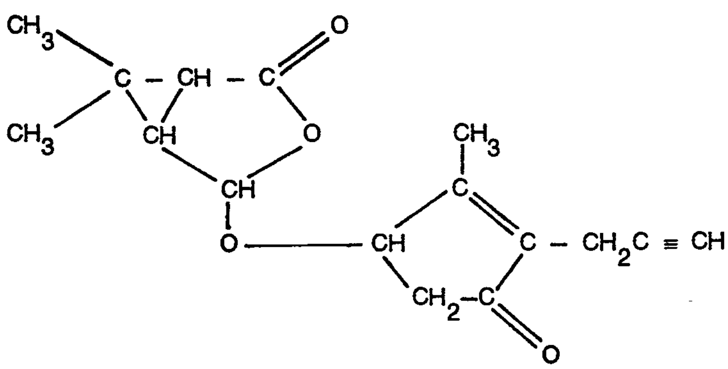

(II)